(19) Europäisches Patentamt
European Patent Office
Office européen des brevets

(11) **EP 3 506 961 B1**

(12) **EUROPÄISCHE PATENTSCHRIFT**

(45) Veröffentlichungstag und Bekanntmachung des Hinweises auf die Patenterteilung:
**12.08.2020** Patentblatt **2020/33**

(51) Int Cl.:
*A61M 1/16* (2006.01)        *A61B 5/145* (2006.01)
*A61M 1/36* (2006.01)

(21) Anmeldenummer: **17767726.7**

(22) Anmeldetag: **04.09.2017**

(86) Internationale Anmeldenummer:
**PCT/EP2017/001053**

(87) Internationale Veröffentlichungsnummer:
**WO 2018/041406 (08.03.2018 Gazette 2018/10)**

(54) **VERFAHREN UND VORRICHTUNG ZUR BESTIMMUNG DER KÖRPERTEMPERATUR EINES PATIENTEN**

METHOD AND DEVICE FOR DETERMINING THE BODY TEMPERATURE OF A PATIENT

PROCÉDÉ ET DISPOSITIF POUR DÉTERMINER LA TEMPÉRATURE CORPORELLE D'UN PATIENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.09.2016 DE 102016010722**

(43) Veröffentlichungstag der Anmeldung:
**10.07.2019** Patentblatt **2019/28**

(73) Patentinhaber: **Fresenius Medical Care Deutschland GmbH**
**61352 Bad Homburg (DE)**

(72) Erfinder: **MAIERHOFER, Andreas**
**97422 Schweinfurt (DE)**

(74) Vertreter: **Herrmann, Uwe**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
**EP-A2- 0 265 795      EP-A2- 0 773 035**
**WO-A1-03/055544      WO-A2-2016/025268**

## Beschreibung

Die vorliegende Erfindung betrifft ein Verfahren und eine Vorrichtung zur Bestimmung der Körpertemperatur oder einer damit korrelierten Temperatur eines an einen extrakorporalen Blutkreislauf angeschlossenen Patienten, wobei der extrakorporale Blutkreislauf über einen Wärmeübertrager verfügt, der auf einer Seite von Blut und auf der anderen Seite von einem Wärmeträgermedium durchströmt wird.

Aus dem Stand der Technik ist es bekannt, vor und während der Dialysebehandlung die Körpertemperatur eines Dialysepatienten zu messen. Diese ist ein wichtiger Indikator für den physiologischen Zustand des Patienten, beispielsweise während einer Hämodialysebehandlung oder Hämodiafiltrationsbehandlung.

Die Körpertemperatur des Patienten vor der Behandlung wird im Wesentlichen durch dessen Biorhythmus bestimmt und kann ggf. durch Erkrankungen, wie beispielsweise Entzündungen erhöht sein.

Die Dialysebehandlung selbst kann durch unterschiedliche Faktoren ebenfalls die Körpertemperatur beeinflussen. So führt beispielsweise eine Anregung des Katabolismus während der Behandlung ebenso wie die Reduzierung des Blutvolumens durch Ultrafiltration zu einem leichten Anstieg der Körpertemperatur. Stärkere und schnellere Anstiege der Temperatur können beispielsweise durch Unverträglichkeitsreaktionen auf die Dialysebehandlung bedingt sein, die durch den Kontakt des Blutes mit Komponenten des Dialysators oder des übrigen extrakorporalen Systems oder durch intradialytische Medikamentengaben hervorgerufen werden.

Als Gegenreaktion des Körpers kann es dazu kommen, dass bei Überschreiten bestimmter Temperaturschwellen durch physiologische Regelmechanismen eine stärkere Verlagerung der Blutzirkulation in die Körperperipherie erfolgt, um den Körper abzukühlen. Diese Verlagerung führt jedoch gleichzeitig zu einem Absinken des zentralen Blutdrucks, was zusammen mit dem Volumenentzug durch die während der Behandlung stattfindende Ultrafiltration zu einem Blutdruckabfall führen kann.

Vor diesem Hintergrund ist die Messung der Körpertemperatur vor und während der Dialysebehandlung von diagnostischem Wert. Abgesehen davon können durch die Messung der Körpertemperatur kritische Zustände während der Dialyse erkannt und Gegenmaßnahmen ergriffen werden. Denkbar ist beispielsweise die Steuerung der Dialysattemperatur mit der Körpertemperatur als Eingangsgröße, um eine sichere und komfortable Dialysebehandlung durchführen zu können.

Ein Nachteil der Messung der Körpertemperatur besteht in dem damit verbundenen apparativen und personellen Zusatzaufwand.

In der klinischen Praxis erfolgt die Messung der Körpertemperatur mit einem Fieberthermometer, entweder durch Kontakt oder als Infrarotmessung. Die Auswertung des gemessenen Wertes erfolgt durch das Pflegepersonal, das aus dem Messwert Rückschlüsse auf den Zustand des Patienten zieht und bei Bedarf die Dialysattemperatur anpasst. Derartige Messungen finden im Allgemeinen nur statt, wenn der Patient bestimmte Symptome zeigt, so dass intradialytische Änderungen der Körpertemperatur nicht oder nur selten bemerkt werden.

Aus dem Stand der Technik sind auch automatisierte kontinuierliche Messungen bekannt, bei denen am arteriellen Blutschlauch des extrakorporalen Blutkreislaufs gemessen wird und bei denen durch die Messung des Temperaturabfalls zwischen der Punktionsstelle und der Messstelle auf die Körpertemperatur geschlossen wird. Die dafür verwendeten Sensoren sind als Zusatzteile verfügbar, was mit zusätzlichen Anschaffungskosten und einem Zusatzaufwand beim Einlegen des Schlauchsystems in die Sensorköpfe einhergeht.

Aus der WO 2016/025268 A2 ist ein Verfahren gemäß dem Oberbegriff des Anspruchs 1 bekannt. Weiterhin zeigen die EP 0 265 795 A2, die EP 0 773 035 A2 und die WO 03/055544 A1 Verfahren zur Bestimmung der Körpertemperatur von Patienten während der extrakorporalen Blutbehandlung.

Der vorliegenden Erfindung liegt daher die Aufgabe zugrunde, ein Verfahren und eine Vorrichtung anzugeben, mittels derer es möglich ist, ohne zusätzliche Hardware allein mit den in der Hydraulik, d.h. z.B. allein mit den im Dialysatsystem eines Dialysegerätes bereits vorhandenen Elementen, insbesondere mittels der dort ohnehin vorhandenen Sensoren den Absolutwert und/oder den Verlauf der Körpertemperatur des Patienten zu erfassen bzw. zu approximieren.

An dieser Stelle wird darauf hingewiesen, dass das erfindungsgemäße Verfahren und die erfindungsgemäße Vorrichtung nicht auf die Dialyse beschränkt sind, sondern auch andere Behandlungsverfahren für Patienten umfassen, bei denen extrakorporale Blutkreisläufe zum Einsatz kommen, wie beispielsweise bei der therapeutischen Hypothermie, bei der das Blut auf einen bestimmten Temperaturwert unterhalb des physiologischen Wertes abgekühlt wird.

Diese genannte Aufgabe wird durch ein Verfahren mit den Merkmalen des Anspruchs 1 sowie durch eine Vorrichtung mit den Merkmalen des Anspruchs 6 gelöst.

Danach ist vorgesehen, dass die eingangsseitige Temperatur ($T_{di}$) des Wärmeträgermediums, wie z.B. des Dialysats oder eines Temperierfluids zur Temperatureinstellung des Blutes, am Eingang des im extrakorporalen Blutkreislauf angeordneten Wärmeübertragers (im Folgenden auch als "Wärmetauscher" bezeichnet) sowie die ausgangsseitige Temperatur ($T_{do}$) des Wärmeträgermediums am Ausgang des Wärmeübertragers sowie der Volumenstrom des Wärmeträgermediums ($Q_d$) gemessen wird und dass die eingangsseitige Temperatur ($T_{bi}$) des Blutes am Eingang des Wärmeübertragers nach der Beziehung

$$T_{bi} = T_{di} + (Q_d/D)\,(T_{do} - T_{di}) \qquad (1)$$

bestimmt wird, wobei es sich bei der Größe D um eine für den Wärmeübergang durch den Wärmeübertrager charakteristische Größe handelt. Aus Formel (1) ist ersichtlich, dass keine blutseitige Messung vorgenommen werden muss, um den Temperaturwert für das Blut zu bestimmen. Ausreichend sind die Messungen der Temperatur des Dialysats oder eines sonstigen Wärmeträgermediums vor und nach dem Dialysator / Wärmeübertrager sowie die Messung des Volumenstrom des Dialysats bzw. des Wärmeträgermediums.

Gemäß dem Fourier-Gesetz ist der Wärmestrom J zwischen zwei Kompartimenten proportional zu deren Temperaturdifferenz. Im Falle eines Dialysators werden diese Kompartimente einerseits durch den blutdurchströmten Raum und andererseits durch den dialysatdurchströmten Raum gebildet. Üblicherweise besteht der blutdurchströmte Raum aus den Innenräumen eines Hohlfaserbündels und der dialysatdurchströmte Raum aus dem dieses umgebenden Raum.

An dieser Stelle wird darauf hingewiesen, dass der Ausdruck, dass der Wärmeübertrager auf einer Seite von Blut und auf der anderen Seite von einem Wärmeträgermedium durchströmt wird, den Fall umfasst, dass für Blut und für das Wärmeträgermedium jeweils genau ein Kompartiment oder Raum vorgesehen sind sowie auch den Fall, dass eine Vielzahl von Kompartimenten bzw. Räumen für das Blut und/oder für das Wärmeträgermedium vorhanden sind.

Im Übrigen wird darauf hingewiesen, dass der im Rahmen der Erfindung verwendete Begriff "ein" oder "eine" sowohl den Fall einschließt, dass genau eines der fraglichen Elemente vorhanden ist, aber auch den Fall, dass eine Mehrzahl der fraglichen Elemente vorhanden sind.

Geht man von einem Dialysator als Wärmeübertrager bzw. Wärmetauscher aus, ergibt sich für den Wärmestrom J: $J = D\,(T_{bi} - T_{di})$, wobei es sich bei $T_{bi}$ um die blutseitige Eingangstemperatur in den Dialysator und bei $T_{di}$ um die dialysatseitige Eingangstemperatur in den Dialysator handelt.

D ist eine Größe, die den Wärmeaustausch bzw. -übergang im Dialysator bzw. Wärmeübertrager beschreibt. D ist abhängig von dem geometrischen Aufbau und von den Materialeigenschaften des Dialysators bzw. Wärmeübertragers, von den Stoffeigenschaften des Bluts und des Dialysats bzw. des Wärmeträgermediums sowie von den Flussraten von Blut und des Dialysats bzw. des Wärmeträgermediums.

Vorstehende und im Folgenden gemachte Bezugnahmen auf den Dialysator und das Dialysat stehen stellvertretend für jeden beliebigen Wärmeübertrager und jedes beliebige Wärmeträgermedium.

Der in das Dialysat eingetragene Wärmestrom muss bei der Vernachlässigung von Abstrahlverlusten mittels des Dialysats auch wieder abtransportiert werden (Energieerhaltung), so dass für J auch gilt: $J = Q_d\,(T_{do} - T_{di})$, wobei es sich bei $T_{do}$ um die dialysatseitige Ausgangstemperatur aus dem Dialysator handelt und bei $Q_d$ um den Dialysatfluss, d.h. um den Volumenstrom des Dialysats durch den Dialysator.

Kombiniert man beide Formeln ergibt sich die obige Beziehung (1):

$$T_{bi} = T_{di} + (Q_d/D)\,(T_{do} - T_{di}) \qquad (1)$$

Somit kann die Bluttemperatur am Einlass des Dialysators bei bekanntem D allein mittels Größen bestimmt werden, die auf der Dialysatseite gemessen werden bzw. dort bekannt sind. Das Fehlen eines Zeichen bzw. eines angegebenen Operators zwischen den Klammerausdrücken $(Q_d/D)$ und $(T_{do} - T_{di})$ ist als Multiplikation der Klammerausdrücke zu verstehen. Dies gilt entsprechend für sämtliche im Rahmen der Erfindung genannte Formeln.

Die Größe D kann bestimmt werden, indem die Methoden angewandt werden, wie sie zur Bestimmung der Dialysance für den diffusiven Stofftransfer in einem Dialysator verwendet werden. Wir verweisen in diesem Zusammenhang auf das Standardwerk: Replacement of Renal Function by Dialysis", 4th edition; chapter 2, "Principles and Biophysics of Dialysis" (Sargent & Gotch), pp. 69-74 und chapter 9, "Hemodialysis Fluid Composition" (C. Ronco et al) pp. 263-266.

Durch das erfindungsgemäße Verfahren und die erfindungsgemäße Vorrichtung lässt sich die Körpertemperatur ermitteln sowie auch eine damit korrelierte Größe, wie beispielsweise die Bluttemperatur im extrakorporalen Kreislauf.

In einer denkbaren Ausgestaltung der Erfindung wird die Größe D bei bekannter Kenngröße $k_0\,A$ des Wärmeübertragers aus der Beziehung

$$D = Q_b\,(e^{\gamma} - 1)/(e^{\gamma} - (Q_b/Q_d)) \qquad (2)$$

mit $\gamma = k_0\,A\,(Q_d - Q_b)/(Q_d Q_b)$

oder

$$D = Q_b (1 - e^{-\gamma})/(1 + (Q_b/Q_d)) \qquad (3)$$

mit $\gamma = k_0 A (Q_d + Q_b)/(Q_d Q_b)$

ermittelt, wobei die Formel (2) für die Durchströmung des Wärmeübertragers durch Blut und Wärmeträgermedium im Gegenstrom und die Formel (3) für die Durchströmung des Wärmeübertragers durch Blut und Wärmeträgermedium im Gleichstrom gilt und wobei die Größe $Q_b$ den Volumenstrom des Blutes durch den Wärmeübertrager darstellt.

Ist die Größe $k_0 A$ des Wärmeübertragers nicht bekannt, so kann D (bzw. über die Gleichungen (2) und (3) auch $k_0 A$) in Analogie zur Bestimmung der diffusen Dialysatorclearance durch Temperaturvariationen auf der Dialysatseite, z.B. in Form von Puls- oder Stufenprofilen bestimmt werden.

Dies setzt voraus, dass der zeitliche Verlauf $T_j$ für einen Temperaturänderungspuls j der Temperatur des Dialysats am Dialysatoreingang und -ausgang gemessen wird und die Fläche unter der Temperaturkurve durch Integralbildung ermittelt wird.

Bei Anwendung des Pulsverfahrens ergibt sich dann für D:

$$D = Q_d (1 - A_{do}/A_{di}) \qquad (4)$$

wobei $A_{do}$ die ggf. durch eine Basislinie korrigierte Fläche unter der Temperaturkurve über die Zeit an der Ausgangsseite des Wärmeübertragers und $A_{di}$ die ggf. durch eine Basislinie korrigierte Fläche unter der Temperaturkurve über die Zeit an der Eingangsseite des Wärmeübertragers darstellt.

Die Flächen $A_{do}$ bzw. $A_{di}$, allgemein $A_j$ sind somit für den jeweiligen Puls: $A_j = \int T_j(t)dt - BL_j$, wobei $BL_j$ eine geeignet bestimmte Basislinie für den Puls j darstellt.

Denkbar ist es ferner, dass die Größe D aus der Beziehung

$$D = f Q_b \qquad (5)$$

ermittelt wird, wobei vorzugsweise vorgesehen ist, dass der Wert f bei 1 liegt oder in der Spanne $1 \pm 0{,}1$ oder $1 \pm 0{,}2$ liegt. Diese Erkenntnis basiert auf der Tatsache, dass der Wärmeaustausch bzw. -übergang im Gegensatz zum Stoffaustausch weitgehend unabhängig von Eigenschaften der die Stoffdiffusion bestimmenden Poren ist und auch ohne direkten Kontakt der beiden Flüssigkeiten untereinander erfolgt. Somit kann D auch vereinfacht als proportional zum Blutfluss $Q_b$ angenommen werden.

In erster Näherung kann für f der Wert 1 verwendet werden.

Auch ist es möglich, den Wert $T_{bi}$ nach Formel (1) zu bestimmen und mit Messwerten der Bluttemperatur zu vergleichen. Somit kann f als Fitfaktor für verschiedene Typen von Dialysatoren bzw. Wärmetauscher bestimmt und zur Verwendung in einer Recheneinheit des Dialyse- bzw. Behandlungsgerätes tabelliert abgelegt werden.

Denkbar ist es weiterhin, dass die Bluttemperatur im extrakorporalen Kreislauf zum Zwecke der späteren Abschätzung der Blut- bzw. Körpertemperatur gemessen wird und durch Regression, vorzugsweise durch lineare Regression eine Beziehung zwischen dem gemessenen Wert ($T_{art}$) (Bluttemperatur in der arteriellen Leitung des extrakorporalen Blutkreislauf) und dem nach Formel (1) bestimmten Wert $T_{bi}$ hergestellt wird. So kann beispielsweise eine Regressionsgleichung der Art

$$T_{art} = a T_{bi} + b \qquad (6)$$

erstellt werden, wobei die Koeffizienten a und b z.B. durch eine least-square-Methode bestimmt werden können.

Diese Beziehung kann dann verwendet werden, um aus den nach Formel (1) bestimmten Werten $T_{bi}$ den Wert für $T_{art}$ zu ermitteln. Dieser Wert kann dann beispielsweise dazu herangezogen werden, die Körpertemperatur $T_{Körper}$ zu bestimmen.

Dies kann beispielsweise nach der Beziehung

$$T_{Körper} = T_{Umgebung} + (T_{art} - T_{Umgebung}) \exp(\alpha L/Q_b) \qquad (7)$$

erfolgen, wobei es sich bei $\alpha$ um die thermische Leitfähigkeit pro Längeneinheit des Schlauchstückes des extrakorporalen Kreislaufes zwischen dem arteriellen Gefäßzugang und dem Wärmeübertrager, bei $T_{Umgebung}$ um die Umgebungstemperatur und bei L um die Länge dieses Schlauchstückes handelt.

Aus Gleichung (7) ergibt sich, dass bei hohen Werten für $Q_b$ die Körpertemperatur im Wesentlichen der Temperatur $T_{art}$ entspricht.

In einer denkbaren Ausgestaltung der Erfindung ist vorgesehen, dass die ermittelte Körpertemperatur oder der damit korrelierte Wert herangezogen wird, um darauf basierend die Dialysattemperatur einzustellen. Ist der z.B. ermittelte Wert der Körpertemperatur zu gering, wird die Dialysattemperatur angehoben und umgekehrt. Denkbar ist auch ein Regelkreis, bei dem die ermittelte Körpertemperatur oder der damit korrelierte Wert die Regelgröße darstellen und mittels der Dialysattemperatur als Stellgröße auf einem Sollwert oder in einem Sollwertbereich gehalten werden. Dies gilt für das erfindungsgemäße Verfahren sowie auch für die erfindungsgemäße Vorrichtung.

Denkbar ist es ferner, dass die Dialysattemperatur als Eingangsgröße für beliebige Regelungen und Profile genutzt werden kann.

Des Weiteren kann vorgesehen sein, dass die Ausgabe eines Alarms erfolgt, wenn die Körpertemperatur oder ein damit korrelierter Wert einen Grenzwert oder einen Grenzwertbereich über- oder unterschreitet. Entsprechendes gilt nicht nur für die Absolutwert der Körpertemperatur oder des damit korrelierten Wertes, sondern auch für die Änderung der Körpertemperatur bzw. des damit korrelierten Wertes gegenüber einem anderen Wert, insbesondere gegenüber der Ausgangstemperatur, d.h. von der der Erfindung ist auch eine Deltaüberwachung umfasst. Schließlich gilt dies entsprechend auch für die Erfassung der Geschwindigkeit der Änderung der Körpertemperatur oder des damit korrelierten Wertes. Übersteigt oder unterschreitet diese einen Grenzwert oder Grenzwertbereich kann ebenfalls ein Alarm ausgegeben werden.

Unter dem Begriff "Alarm" ist jede beliebige durch einen Nutzer oder das medizinische Personal wahrnehmbare Information zu verstehen, wie beispielsweise ein Alarmton im engeren Sinne oder eine Mitteilung in Textform etc.

Die vorliegende Erfindung betrifft des Weiteren eine Vorrichtung zur Bestimmung der Körpertemperatur oder einer damit korrelierten Temperatur eines Patienten mit einem extrakorporalen Blutkreislauf, wobei der extrakorporale Blutkreislauf über einen Wärmeübertrager verfügt, der auf einer Seite von Blut und auf der anderen Seite von einem Wärmeträgermedium durchströmt wird, wobei die Vorrichtung Messfühler aufweist, die angeordnet sind, die eingangsseitige Temperatur ($T_{di}$) des Wärmeträgermediums am Eingang des Wärmeübertragers sowie die ausgangsseitige Temperatur ($T_{do}$) des Wärmeträgermediums am Ausgang des Wärmeübertragers sowie den Volumenstrom des Wärmeträgermediums ($Q_d$) zu messen und wobei die Vorrichtung eine Berechnungseinheit aufweist, die ausgebildet ist, die eingangsseitige Temperatur ($T_{bi}$) des Blutes am Eingang des Wärmeübertragers nach der Beziehung

$$T_{bi} = T_{di} + (Q_d/D)\,(T_{do} - T_{di}) \qquad (1)$$

zu bestimmen, wobei es sich bei der Größe D um eine für den Wärmeübergang durch den Wärmeübertrager charakteristische Größe handelt.

Denkbar ist es, dass es sich bei dem Wärmeübertrager um einen Dialysator handelt und/oder dass es sich bei der mit der Körpertemperatur korrelierten Temperatur um die Temperatur des Blutes in dem extrakorporalen Kreislauf handelt.

Weiterhin kann vorgesehen sein, dass die Vorrichtung Berechnungsmittel aufweist, die ausgebildet sind, die Größe D bei bekannter Kenngröße $k_0$ A des Wärmeübertragers aus der Beziehung

$$D = Q_b\,(e^{\gamma} - 1)/(e^{\gamma} - (Q_b/Q_d)) \qquad (2)$$

mit $\gamma = k_0\,A\,(Q_d - Q_b)/(Q_d Q_b)$
oder

$$D = Q_b\,(1 - e^{-\gamma})/(1 + (Q_b/Q_d)) \qquad (3)$$

mit $\gamma = k_0\,A\,(Q_d + Q_b)/(Q_d Q_b)$
zu ermitteln, wobei die Formel (2) für die Durchströmung des Wärmeübertragers im Gegenstrom und die Formel (3) für die Durchströmung des Wärmeübertragers im Gleichstrom gilt und wobei die Größe $Q_b$ den Volumenstrom des Blutes durch den Wärmeübertrager darstellt.

Die Vorrichtung kann Berechnungsmittel zur Ermittlung der Größe D aufweisen, die ausgebildet sind, auf der Seite des Wärmeträgermediums eine Temperaturvariation zu generieren und den zeitlichen Verlauf der Temperatur des Wärmeträgermediums am Eingang und am Ausgang bzw. an den Messstellen über die Zeit zu integrieren und aus der Beziehung

$$D = Q_d \, (1 - A_{do}/A_{di}) \qquad (4)$$

die Größe D zu bestimmen, wobei $A_{do}$ die ggf. durch eine Basislinie korrigierte Fläche unter der Temperaturkurve über die Zeit an der Ausgangsseite des Wärmeübertragers und $A_{di}$ die ggf. durch eine Basislinie korrigierte Fläche unter der Temperaturkurve über die Zeit an der Eingangsseite des Wärmeübertragers darstellt.

Weiterhin kann die Vorrichtung Berechnungsmittel aufweisen, die ausgebildet sind, die Größe D aus der Beziehung

$$D = f \, Qb \qquad (5)$$

zu ermitteln, wobei vorzugsweise vorgesehen ist, dass der Wert f bei 1 liegt oder in der Spanne 1 ± 0,1 oder 1 ± 0,2 liegt.

In einer weiteren Ausgestaltung der Erfindung ist vorgesehen, dass ein Messfühler vorgesehen ist, um die Bluttemperatur im extrakorporalen Kreislauf zum Zwecke der Ermittlung einer Korrelation zwischen berechneten und tatsächlichen Werten zu messen und dass Regressionsmittel vorgesehen sind, die ausgebildet sind, durch Regression, vorzugsweise durch lineare Regression eine Regressionsgleichung zwischen dem gemessenen Wert ($T_{art}$) und dem nach Formel (1) bestimmten Wert herzustellen.

Diese kann beispielsweise

$$T_{art} = a \, T_{bi} + b \qquad (6)$$

lauten

Weiterhin ist es denkbar, dass die Vorrichtung Berechnungsmittel aufweist, die ausgebildet sind, aus der Regressionsgleichung $T_{art}$ zu berechnen und die Körpertemperatur nach der Beziehung

$$T_{Körper} = T_{Umgebung} + (T_{art} - T_{Umgebung}) \, exp(\alpha L/Q_b) \qquad (7)$$

zu bestimmen, wobei es sich bei $\alpha$ um die thermische Leitfähigkeit pro Längeneinheit des Schlauchstückes des extrakorporalen Kreislaufes zwischen dem arteriellen Gefäßzugang und dem Wärmeübertrager und bei L um die Länge dieses Schlauchstückes handelt.

Wie oben ausgeführt, können Einstellmittel vorhanden sein, mittels derer die Dialysattemperatur veränderbar ist.

Diese bzw. ein Regler können als Eingangsgröße die ermittelte Körpertemperatur oder den damit korrelierten Wert aufweisen und in Abhängigkeit davon die Dialysattemperatur einstellen, die als Stellgröße dient. Dabei kann ein Regler vorgesehen sein, dem die ermittelte Körpertemperatur oder der damit korrelierte Wert als Istwerte zugeführt werden und der diese mittels der Dialysattemperatur auf einen Sollwert oder in einen Sollwertbereich einregelt.

Des Weiteren kann die Vorrichtung wenigstens eine Alarmvorrichtung aufweisen, die geeignet ist, einen Alarm auszugeben, wenn die Körpertemperatur oder ein damit korrelierter Wert einen Grenzwert oder einen Grenzwertbereich über- oder unterschreitet. Entsprechendes gilt nicht nur für den Absolutwert der Körpertemperatur oder des damit korrelierten Wertes, sondern auch für die Änderung der Körpertemperatur bzw. des damit korrelierten Wertes gegenüber einem anderen Wert, insbesondere gegenüber der Ausgangstemperatur, d.h. von der der Erfindung ist auch eine Deltaüberwachung umfasst. Schließlich gilt dies entsprechend auch für die Erfassung der Geschwindigkeit der Änderung der Körpertemperatur oder des damit korrelierten Wertes. Übersteigt oder unterschreitet diese einen Grenzwert oder Grenzwertbereich kann mittels der Alarmvorrichtung ebenfalls ein Alarm ausgegeben werden.

Unter dem Begriff "Alarm" ist jede beliebige durch einen Nutzer oder das medizinische Personal wahrnehmbare Information zu verstehen, wie beispielsweise ein Alarmton im engeren Sinne oder eine Mitteilung in Textform etc.

Alternativ oder zusätzlich kann die Vorrichtung Überwachungsmittel zur Überwachung eines oder mehrerer der genannten Werte (Absolutwert der Körpertemperatur oder des damit korrelierten Wertes; dessen Differenz gegenüber einem Bezugswert; dessen Änderungsgeschwindigkeit) aufweisen.

Die vorliegende Erfindung betrifft des Weiteren ein Blutbehandlungsgerät, insbesondere ein Dialysegerät, das sich dadurch auszeichnet, dass das Blutbehandlungsgerät mit einer Vorrichtung nach einem der Ansprüche 8 bis 14

# EP 3 506 961 B1

ausgeführt ist und/oder Mittel zur Durchführung eines Verfahrens nach einem der Ansprüche 1 bis 7 aufweist.

Weitere Einzelheiten und Vorteile der Erfindung werden anhand eines in der Zeichnung dargestellten Ausführungsbeispiels näher erläutert.

Es zeigen:

Figur 1: einen Vergleich der nach Formel (1) und (5) berechneten Werte der Bluttemperatur (Tbi) und der gemessenen Werte der Temperatur (T_art (BTM)) des Blutes im arteriellen Teil des extrakorporalen Kreislaufs zu Dialysebeginn jeweils in °C,

Figur 2: einen Vergleich der berechneten (dTbi) Temperaturänderung und der gemessenen Änderung der arteriellen Bluttemperatur (dTart (BTM) während der Dialyse und

Figur 3: eine schematische Darstellung eines Dialysegerätes gemäß der Erfindung.

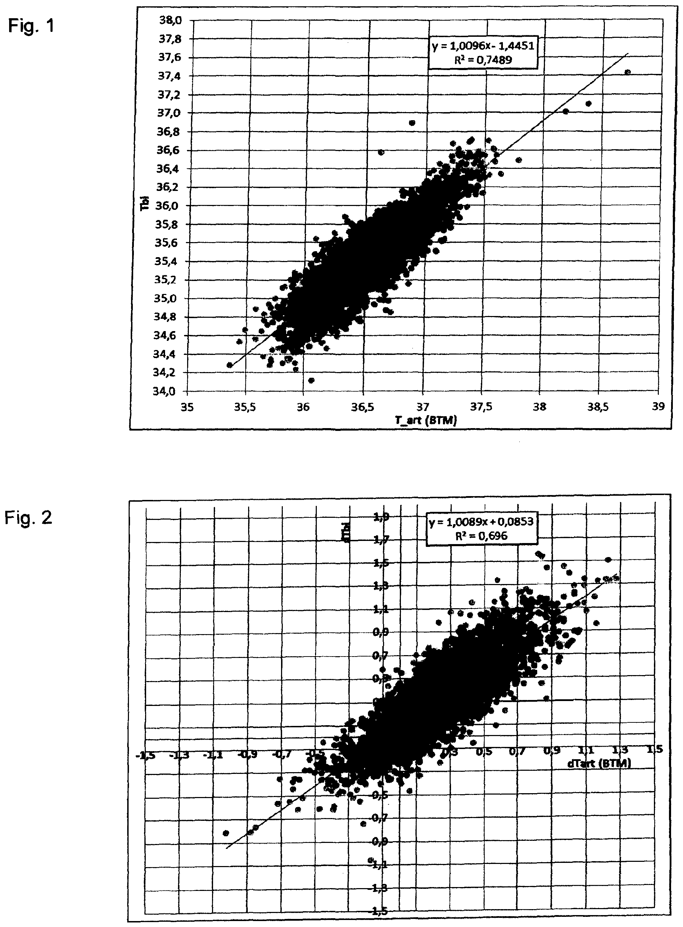

Figur 1 zeigt einen Vergleich berechneter Werte und gemessener Werte für die arterielle Bluttemperatur eines Patienten zu Dialysebeginn auf der Datenbasis von 5.000 Werten bzw. Behandlungen. Der Blutfluss $Q_b$ betrug 350 - 450 ml/min und der Dialysatfluss $Q_d$ betrug 500 - 700 ml/min. Der Wert für D wurde gemäß Formel (5) mit f = 1,1 berechnet.

Figur 1 verdeutlicht eine gute Korrelation zwischen der erfindungsgemäßen dialysatseitigen Berechnung und der blutseitigen Messung der Temperatur, wobei die Standardabweichung der Differenz der beiden Methoden bei 0,19 °C liegt. Die systematische Abweichung lässt sich durch eine Korrektur mit Hilfe der aus der Regression bestimmten Koeffizienten gemäß Formel $T_{art}$ = a $T_{bi}$ + b eliminieren.

Neben einer automatischen Registrierung und Dokumentation der Körpertemperatur zu Behandlungsbeginn kann alternativ oder zusätzlich auch eine automatisierte Überwachung auf das Überschreiten eines Schwellwertes mit entsprechender Information an die Schwester oder den Arzt erfolgen. Dies kann notwendig sein, um z.B. auf eine Entzündung aufmerksam zu machen.

Zusätzlich oder alternativ ist auch denkbar, dass ein Alarm ausgegeben wird, wenn die Körpertemperatur oder deren Differenz zu einem Bezugswert, wie beispielsweise zu dem Ausgangswert der Körpertemperatur bei Behandlungsbeginn, oder wenn deren Änderungsgeschwindigkeit einen Grenzwert oder Grenzwertbereich über- oder unterschreitet.

In dem in Figur 1 gezeigten Beispiel konnte durch die dialysatseitige Messung die Überschreitung einer arteriell gemessenen Temperatur von 37,2 °C mit einer Sensitivität von 82 % und einer Spezifität von 98 % erkannt werden.

Figur 2 zeigt einen Vergleich von erfindungsgemäß, d.h. durch dialysatseitige Sensoren berechneten Werten und gemessenen Werten für die Änderung der arteriellen Bluttemperatur eines Patienten auf der Datenbasis von 5.000 Werten bzw. Behandlungen während der Behandlung. Der Blutfluss $Q_b$ betrug 350 - 450 ml/min und der Dialysatfluss $Q_d$ betrug 500 - 700 ml/min. Der Wert für D wurde gemäß Formel (5) mit f = 1,1 berechnet. Auch hier zeigt sich eine gute Korrelation zwischen der dialysatseitigen Berechnung gemäß der Erfindung und der blutseitigen Messung, wobei die Standardabweichung der Differenz der Methoden 0,17 °C beträgt.

Erfindungsgemäß ist somit auch eine Überwachung der intradialytischen Änderung der Patiententemperatur möglich.

Wie eingangs ausgeführt, kann ein Temperaturanstieg auf eine Unverträglichkeitsreaktion hindeuten oder es kann durch einen durch beliebige Ursachen hervorgerufenen Temperaturanstieg zu einem Blutdruckabfall kommen.

Durch die vorzugsweise automatisch ablaufende Erfassung der Temperaturänderung kann das Dialysegerät automatisch eine Warnung vor eventuellen Unverträglichkeitsreaktionen ausgeben, d.h. entsprechende Ausgabemittel aufweisen, vor ggf. drohenden Blutdruckabfällen warnen, eine Blutdruckmessung automatisiert auslösen bzw. deren Frequenz erhöhen oder die Dialysattemperatur absenken, um dem Anstieg der Körpertemperatur entgegenzuwirken oder auch eine Mehrzahl der vorgenannten Maßnahmen vornehmen. Das Dialysegerät bzw. sonstige Behandlungsgerät kann Mittel aufweisen, um eine oder mehrere dieser Maßnahmen zu ergreifen.

In dem in Figur 2 gezeigten Beispiel konnte durch die dialysatseitige Messung das Vorliegen eines Temperaturanstiegs von mehr als 0,5 °C mit einer Sensitivität von 73 % und einer Spezifität von 90 % erkannt werden.

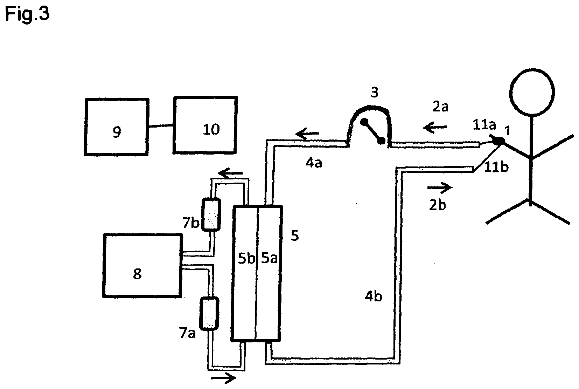

Wie bereits oben ausgeführt, kann die gemäß Formel (1) oder die nach $T_{art}$ = a $T_{bi}$ + b oder die nach Formel (7) bestimmte Temperatur als Eingangsgröße für die Regelung dieser Temperatur auf einen Sollwert oder in einem Sollwertbereich herangezogen werden, wozu die in Figur 3 gezeigte Temperiereinheit 8 verwendet werden kann und wozu das Gerät eine Regelungseinheit 9 aufweist.

Insbesondere das Konstanthalten der Temperatur zu Beginn der Dialyse ist mit dem erfindungsgemäßen Verfahren bzw. der erfindungsgemäßen Vorrichtung ohne wesentliche Genauigkeitseinbußen gegenüber dem auf der Messung mit blutseitigen Sensoren basierenden Verfahren möglich, da hier systematische Abweichungen zwischen der erfindungsgemäßen Vorgehensweise und der Referenz ohne Bedeutung sind.

Vorzugsweise werden erfindungsgemäß zur Temperaturmessung nur Elemente verwendet, die ohnehin an einem Dialysegerät vorhanden sein, was Kostenvorteile mit sich bringt. So kann beispielsweise auf blutseitig angeordnete Temperatursensoren verzichtet werden.

Figur 3 zeigt ein Dialysegerät, mittels dessen das erfindungsgemäße Verfahren durchführbar ist bzw. ein Dialysegerät gemäß der Erfindung. Die Blutpumpe 3 fördert Blut aus dem Gefäßzugang 1 über eine erste arterielle Nadel 11a in Richtung 2a und über eine erste Blutleitung 4a zu der Blutseite 5a eines Dialysators oder sonstigen Wärmeübertragers bzw. Wärmetauschers (beide Begriffe werden im Rahmen der Erfindung synonym verwendet).

Die Blutseite 5a steht über eine oder mehrere Membranen oder sonstigen Trennmittel in thermischen Kontakt mit der Dialysatseite 5b bzw. mit einem zweiten Kompartiment 5b des Dialysators 5.

Handelt es sich um eine Hämodialyse oder Hämodiafiltration handelt es sich bei 5b um die Dialyatseite eines Dialysators, bei einer Vorrichtung zur Bluttemperierung um die von einem Wärme- oder Kühlmittel durchflossene Seite des Wärmetauschers.

Zur Temperierung des Dialysats bzw. des Wärme-/Kühlmittels dient die Temperiereinheit 8 als Stellglied, die Elemente, wie z.B. Heiz- und/oder Kühlelemente, Temperatursensoren und analog oder digital gesteuerte Regelvorrichtungen aufweisen kann.

Die Temperatur der in das zweite Kompartiment 5b fließenden Flüssigkeit wird stromauf und stromab des zweiten Kompartiments 5b mittels der Temperatursensoren 7a, 7b gemessen. Die Temperiereinheit 8 kann durch die Auswerte- und/oder Regeleinheit 9 mit dem Ziel einer Änderung der Bluttemperatur in der zweiten Blutleitung 4b gesteuert werden, mittels derer das Blut durch den venösen Zugang 11b in Richtung 2b zurück zum Patienten gefördert wird.

Abgesehen von den Sensoren 7a, 7b sind mit der Regelungseinheit 9 auch Mittel zur Bestimmung des Flüssigkeitsstroms auf der Blutseite und auf der Sekundärseite, d.h. auf der Dialysatseite verbunden.

Zur Bestimmung der Bluttemperatur in den Leitungsabschnitten 4a und 4b sowie der Körpertemperatur und/oder deren zeitlichen Änderungen im Verlauf der Behandlung werden in der Auswerteeinheit 9 verschiedene, oben näher beschriebene Berechnungen durchgeführt und die Ergebnisse können auf einer Anzeigeeinheit 10 bzw. auch über eine andere Art der Kommunikation, wie z.B. über ein Netzwerk oder eine drahtlose Kommunikation z.B. auf ein Smartphone übermittelt werden.

**Patentansprüche**

1. Verfahren zur Bestimmung der Körpertemperatur oder einer damit korrelierten Temperatur eines an einen extrakorporalen Blutkreislauf angeschlossenen Patienten, wobei der extrakorporale Blutkreislauf über einen Wärmeübertrager verfügt, der auf einer Seite von Blut und auf der anderen Seite von einem Wärmeträgermedium durchströmt wird, und wobei die eingangsseitige Temperatur ($T_{di}$) des Wärmeträgermediums am Eingang des Wärmeübertragers sowie die ausgangsseitige Temperatur ($T_{do}$) des Wärmeträgermediums am Ausgang des Wärmeübertragers sowie der Volumenstrom des Wärmeträgermediums ($Q_d$) gemessen wird,
**dadurch gekennzeichnet,**
**dass** die eingangsseitige Temperatur ($T_{bi}$) des Blutes am Eingang des Wärmeübertragers nach der Beziehung

$$T_{bi} = T_{di} + (Q_d/D)\ (T_{do} - T_{di}) \qquad (1)$$

bestimmt wird, wobei es sich bei der Größe D um eine für den Wärmeübergang durch den Wärmeübertrager charakteristische Größe handelt.

2. Verfahren nach Anspruch 1, **dadurch gekennzeichnet, dass** die Größe D bei bekannter Kenngröße $k_0$ A des Wärmeübertragers aus der Beziehung

$$D = Q_b\ (e^{\gamma} - 1)/(e^{\gamma} - (Q_b/Q_d)) \qquad (2)$$

mit $\gamma = k_0$ A $(Q_d - Q_b)/(Q_d Q_b)$ oder

$$D = Q_b\ (1 - e^{-\gamma})/(1 + (Q_b/Q_d)) \qquad (3)$$

mit $\gamma = k_0$ A $(Q_d + Q_b)/(Q_d Q_b)$
ermittelt wird, wobei die Formel (2) für die Durchströmung des Wärmeübertragers im Gegenstrom und die Formel (3) für die Durchströmung des Wärmeübertragers im Gleichstrom gilt und wobei die Größe $Q_b$ den Volumenstrom des Blutes durch den Wärmeübertrager darstellt.

3. Verfahren nach einem der vorhergehenden Ansprüche, **dadurch gekennzeichnet, dass** die Größe D aus der Beziehung

$$D = f\, Q_b \qquad\qquad (5)$$

ermittelt wird, wobei vorgesehen ist, dass der Wert f bei 1 liegt oder in der Spanne $1 \pm 0{,}1$ oder $1 \pm 0{,}2$ liegt.

4. Verfahren nach einem der vorhergehenden Ansprüche, **dadurch gekennzeichnet, dass** die Bluttemperatur im extrakorporalen Kreislauf gemessen wird und durch lineare Regression eine Beziehung zwischen dem gemessenen Wert ($T_{art}$) und dem nach Formel (1) bestimmten Wert hergestellt wird, nämlich gemäß der Beziehung

$$T_{art} = a\, T_{bi} + b. \qquad\qquad (6),$$

wobei weiterhin vorgesehen ist, dass aus der Regressionsgleichung $T_{art}$ berechnet wird und dass die Körpertemperatur nach der Beziehung

$$T_{Körper} = T_{Umgebung} + (T_{art} - T_{Umgebung})\, \exp(\alpha L / Q_b) \qquad\qquad (7)$$

bestimmt wird, wobei es sich bei $\alpha$ um die thermische Leitfähigkeit pro Längeneinheit des Schlauchstückes des extrakorporalen Kreislaufes zwischen dem arteriellen Gefäßzugang (1) und dem Wärmeübertrager und bei L um die Länge dieses Schlauchstückes handelt.

5. Verfahren nach einem der vorhergehenden Ansprüche, **dadurch gekennzeichnet, dass** die Körpertemperatur oder ein damit korrelierter Wert, deren/dessen Differenz gegenüber einem Bezugswert oder deren/dessen Änderungsgeschwindigkeit überwacht wird, wobei vorgesehen ist, dass bei Über- oder Unterschreiten eines Grenzwertes oder Grenzwerbereiches einer der vorgenannten Größen Alarm gegeben wird.

6. Vorrichtung zur Bestimmung der Körpertemperatur oder einer damit korrelierten Temperatur eines Patienten mit einem extrakorporalen Blutkreislauf, wobei der extrakorporale Blutkreislauf über einen Wärmeübertrager verfügt, der auf einer Seite von Blut und auf der anderen Seite von einem Wärmeträgermedium durchströmt wird, und wobei die Vorrichtung Messfühler aufweist, die angeordnet sind, die eingangsseitige Temperatur ($T_{di}$) des Wärmeträgermediums am Eingang des Wärmeübertragers sowie die ausgangsseitige Temperatur ($T_{do}$) des Wärmeträgermediums am Ausgang des Wärmeübertragers sowie den Volumenstrom des Wärmeträgermediums ($Q_d$) zu messen, **dadurch gekennzeichnet, dass** die Vorrichtung eine Berechnungseinheit aufweist, die ausgebildet ist, die eingangsseitige Temperatur ($T_{bi}$) des Blutes am Eingang des Wärmeübertragers nach der Beziehung

$$T_{bi} = T_{di} + (Q_d / D)\, (T_{do} - T_{di}) \qquad\qquad (1)$$

zu bestimmen, wobei es sich bei der Größe D um eine für den Wärmeübergang durch den Wärmeübertrager charakteristische Größe handelt.

7. Vorrichtung nach Anspruch 6, **dadurch gekennzeichnet, dass** es sich bei dem Wärmeübertrager um einen Dialysator (5) handelt und dass es sich bei der mit der Körpertemperatur korrelierten Temperatur um die Temperatur des Blutes in dem extrakorporalen Kreislauf handelt.

8. Vorrichtung nach einem Ansprüche 6 oder 7, **dadurch gekennzeichnet, dass** die Vorrichtung Berechnungsmittel aufweist, die ausgebildet sind, die die Größe D bei bekannter Kenngröße $k_0\, A$ des Wärmeübertragers aus der Beziehung

$$D = Q_b \, (e^\gamma - 1)/(e^\gamma - (Q_b/Q_d)) \qquad (2)$$

mit $\gamma = k_0 \, A \, (Q_d - Q_b)/(Q_d Q_b)$
oder

$$D = Q_b \, (1 - e^{-\gamma})/(1 + (Q_b/Q_d)) \qquad (3)$$

mit $\gamma = k_0 \, A \, (Q_d + Q_b)/(Q_d Q_b)$
zu ermitteln, wobei die Formel (2) für die Durchströmung des Wärmeübertragers im Gegenstrom und die Formel (3) für die Durchströmung des Wärmeübertragers im Gleichstrom gilt und wobei die Größe $Q_b$ den Volumenstrom des Blutes durch den Wärmeübertrager darstellt.

9. Vorrichtung nach einem der Ansprüche 6 bis 8, **dadurch gekennzeichnet, dass** die Vorrichtung Berechnungsmittel zur Ermittlung der Größe D aufweist, die ausgebildet sind, auf der Seite des Wärmeträgermediums eine Temperaturvariation vorzunehmen und den zeitlichen Verlauf der Temperatur des Wärmeträgermediums am Eingang und am Ausgang über die Zeit zu integrieren und aus der Beziehung

$$D = Q_d \, (1 - A_{do}/A_{di}) \qquad (4)$$

die Größe D zu bestimmen, wobei $A_{do}$ die ggf. durch eine Basislinie korrigierte Fläche unter der Temperaturkurve über die Zeit an der Ausgangsseite des Wärmeübertragers und $A_{di}$ die ggf. durch eine Basislinie korrigierte Fläche unter der Temperaturkurve über die Zeit an der Eingangsseite des Wärmeübertragers darstellt.

10. Vorrichtung nach einem der Ansprüche 6 bis 9, **dadurch gekennzeichnet, dass** die Vorrichtung Berechnungsmittel aufweist, die ausgebildet sind, die Größe D aus der Beziehung

$$D = f \, Qb \qquad (5)$$

zu ermitteln, wobei vorgesehen ist, dass der Wert f bei 1 liegt oder in der Spanne $1 \pm 0{,}1$ oder $1 \pm 0{,}2$ liegt.

11. Vorrichtung nach einem der Ansprüche 6 bis 10, **dadurch gekennzeichnet, dass** die Vorrichtung Überwachungsmittel aufweist, die ausgeführt sind, die Körpertemperatur oder einen damit korrelierten Wert, deren/dessen Differenz gegenüber einem Bezugswert oder deren/dessen Änderungsgeschwindigkeit zu überwachen, wobei vorgesehen ist, dass bei Über- oder Unterschreiten eines Grenzwertes oder Grenzwerbereiches eines der vorgenannten Werte Alarm gegeben wird.

12. Vorrichtung nach einem der Ansprüche 6 bis 11, **dadurch gekennzeichnet, dass** die Vorrichtung ausgebildet ist, aus einem berechneten bzw. bekannten Wert für $T_{art}$ die Körpertemperatur nach der Beziehung

$$T_{K\ddot{o}rper} = T_{Umgebung} - (T_{art} - T_{Umgebung}) \, \exp(\alpha L/Q_b) \qquad (7)$$

zu bestimmen, wobei es sich bei $\alpha$ um die thermische Leitfähigkeit pro Längeneinheit des Schlauchstückes des extrakorporalen Kreislaufes zwischen dem arteriellen Gefäßzugang (1) und dem Wärmeübertrager und bei L um die Länge dieses Schlauchstückes handelt.

13. Blutbehandlungsgerät umfassend eine Vorrichtung nach einem der Ansprüche 6 bis 12.

14. Blutbehandlungsgerät nach Anspruch 13, **dadurch gekennzeichnet, dass** es sich bei dem Blutbehandlungsgerät um ein Dialysegerät und bei dem Wärmeübertrager der Vorrichtung um einen Dialysator (5) handelt.

15. Blutbehandlungsgerät nach Anspruch 14, **dadurch gekennzeichnet, dass** die Vorrichtung eine Regelungseinheit (9) aufweist, die ausgebildet ist, die Körpertemperatur eines mit dem Dialysegerät behandelten Patienten oder eine damit korrelierte Temperatur auf einen Sollwert oder in einen Sollwertbereich zu regeln, wobei die Regelungseinheit (9) einen Eingang für die berechnete Temperatur als Istwert aufweist und ausgebildet ist, diesen mit einem Sollwert oder Sollwertbereich zu vergleichen, und wobei die Regelungseinheit (9) mit einer Temperiereinheit (8) in Verbindung steht, die ausgebildet ist, die als Stellgröße dienende Temperatur eines den Dialysator (5) durchströmenden Dialysats zu verändern, sofern der Istwert nicht dem Sollwert entspricht oder nicht in dem Sollwertbereich liegt.

**Claims**

1. Method of determining the body temperature or of a temperature correlated therewith of a patient connected to an extracorporeal blood circuit, wherein the extracorporeal blood circuit has a heat exchanger which is flowed through by blood at one side and by a heat carrier medium at the other side, and wherein the temperature ($T_{di}$) of the heat carrier medium at the inlet side is measured at the inlet of the heat exchanger and the temperature ($T_{do}$) of the heat carrier medium at the outlet side is measured at the outlet of the heat exchanger and the volume flow of the heat carrier medium ($Q_d$) is measured;
   **characterized in that**
   the temperature ($T_{bi}$) of the blood at the inlet side is measured at the inlet of the heat exchanger in accordance with the relationship

$$T_{bi} = T_{di} + (Q_d/D)\,(T_{do} - T_{di}) \qquad (1)$$

   where the value D is a value characteristic of the heat transfer by the heat exchanger.

2. Method in accordance with claim 1 or claim 2, **characterized in that** the value D is determined, with a known characteristic $k_0\,A$ of the heat exchanger, from the relationship

$$D = Q_b\,(e^{\gamma} - 1)/(e^{\gamma} - (Q_b/Q_d)) \qquad (2)$$

   where $\gamma = k_0\,A\,(Q_d - Q_b)/(Q_d Q_b)$
   or

$$D = Q_b\,(1 - e^{-\gamma})/(1 + (Q_b/Q_d)) \qquad (3)$$

   where $\gamma = k_0\,A\,(Q_d + Q_b)/(Q_d Q_b)$
   with the formula (2) applying to the flowing through of the heat exchanger in a counter flow and the formula (3) applying to the flowing through of the heat exchanger in coflow, and with the value $Q_b$ representing the volume flow of the blood through the heat exchanger.

3. Method in accordance with one of the preceding claims, **characterized in that** the value D is determined from the relationship

$$D = f\,Q_b \qquad (5)$$

   where provision is made that the value f is 1 or is in the range $1 \pm 0.1$ or $1 \pm 0.2$.

4. Method in accordance with one of the preceding claims, **characterized in that** the blood temperature in the extracorporeal circuit is measured and a relationship is established between the measured value ($T_{art}$) and the value determined in accordance with formula (1) by linear regression, namely in accordance with the relationship

$$T_{art} = a\,T_{bi} + b \qquad (6)$$

   wherein it is further provided that $T_{art}$ is calculated from the regression equation and that the body temperature is determined in accordance with the relationship

$$T_{\text{Körper}} = T_{\text{Umgebung}} + (T_{\text{art}} - T_{\text{Umgebung}})\, \exp(\alpha L/Q_b) \qquad (7)$$

where $\alpha$ is the thermal conductivity per length unit of the hose piece of the extracorporeal circuit between the arterial vessel port (1) and the heat exchanger and L is the length of this hose piece.

5. Method in accordance with one of the preceding claims, **characterized in that** the body temperature or a value correlated therewith, its difference with respect to a reference value and/or its change speed is monitored, with provision being made that an alarm is output on an exceeding or falling below of a limit value or limit value range of one of the aforesaid values.

6. Apparatus for determining the body temperature or a temperature correlated therewith of a patient having an extracorporeal blood circuit, wherein the extracorporeal blood circuit has a heat exchanger which is flowed through by blood at one side and by a heat carrier medium at the other side; and wherein the apparatus has measurement sensors which are arranged to measure the temperature ($T_{di}$) of the heat carrier medium at the inlet side at the inlet of the heat exchanger and to measure the temperature ($T_{do}$) of the heat carrier medium at the outlet side at the outlet of the heat exchanger and to measure the volume flow of the heat carrier medium (Qd);
**characterized in that**
the apparatus has a calculation unit which is configured to determine the temperature ($T_{bi}$) of the blood at the inlet side at the inlet of the heat exchanger in accordance with the relationship

$$T_{bi} = T_{di} + (Q_d/D)\,(T_{do} - T_{di}) \qquad (1)$$

where the value D is a value characteristic of the heat transfer by the heat exchanger.

7. Apparatus in accordance with Claim 6, **characterized in that** the heat exchanger is a dialyzer (5) and/or that the temperature correlated with the body temperature is the temperature of the blood in the extracorporeal circuit.

8. Apparatus in accordance with one of Claims 6 or 7, **characterized in that** the apparatus comprises calculation means which are configured to determine the value D, with a known characteristic $k_0$ A of the heat exchanger, from the relationship

$$D = Q_b\,(e^{\gamma} - 1)/(e^{\gamma} - (Q_b/Q_d)) \qquad (2)$$

where $\gamma = k_0\,A\,(Q_d - Q_b)/(Q_d Q_b)$
or

$$D = Q_b\,(1 - e^{-\gamma})/(1 + (Q_b/Q_d)) \qquad (3)$$

where $\gamma = k_0\,A\,(Q_d + Q_b)/(Q_d Q_b)$
with the formula (2) applying to the flowing through of the heat exchanger in a counter flow and the formula (3) applying to the flowing through of the heat exchanger in coflow, and with the value $Q_b$ representing the volume flow of the blood through the heat exchanger.

9. Apparatus in accordance with one of the claims 6 to 8, **characterized in that** the apparatus comprises calculation means for determining the value D which are configured to carry out a temperature change at the side of the heat carrier medium and to integrate the time development of the temperature of the heat carrier medium at the inlet and at the outlet over time and to determine the value D from the relationship

$$D = Q_d\,(1 - A_{do}/A_{di}) \qquad (4)$$

where $A_{do}$ represents the area, optionally corrected by a baseline, below the temperature curve over time at the outlet side of the heat exchanger and $A_{di}$ represents the area, optionally corrected by a baseline, below the temperature curve over time at the inlet side of the heat exchanger.

10. Apparatus in accordance with one of claims 6 to 9, **characterized in that** the apparatus comprises calculation means which are configured to determine the value D from the relationship

$$D = f\, Qb \qquad (5)$$

where provision is preferably made that the value f is 1 or is in the range $1 \pm 0.1$ or $1 \pm 0.2$.

11. Apparatus in accordance with one of Claims 6 to 10, **characterized in that** the apparatus has monitoring means which are designed to monitor the body temperature or a value correlated therewith or its difference with respect to a reference value and/or its change speed, with provision being made that an alarm is output on an exceeding or falling below of a limit value or limit value range of one of the aforesaid values.

12. Apparatus in accordance with one of Claims 6 to 11, **characterized in that** the apparatus is configured to determine the body temperature from a calculated or known value for $T_{art}$ in accordance with the relationship

$$T_{K\ddot{o}rper} = T_{Umgebung} - (T_{art} - T_{Umgebung})\, \exp(\alpha L/Qb) \quad (7)$$

where $\alpha$ is the thermal conductivity per length unit of the hose piece of the extracorporeal circuit between the arterial vessel port (1) and the heat exchanger and L is the length of this hose piece.

13. Blood treatment device, comprising an apparatus according to one of Claims 6 to 12.

14. Blood treatment device in accordance with Claim 13, **characterized in that** the blood treatment device is a dialysis machine and the heat exchanger of the apparatus is a dialyzer (5).

15. Apparatus in accordance with Claim 14, **characterized in that** the apparatus has a regulation unit (9) which is configured to regulate the body temperature of the patient treated by means of the dialyzer or a temperature correlated therewith to a desired value or into a desired value range, wherein the regulation unit (9) has an input for the calculated temperature as an actual value and is configured to compare it to a desired value or desired value range, and wherein the regulation unit (9) is connected to a temperature control unit (8), which is configured to change the temperature of the dialysate serving as an adjustment value if the actual value does not correspond to the desired value or if it does not lie in the desired value range.

**Revendications**

1. Procédé servant à définir la température corporelle ou une température en corrélation avec celle-ci d'un patient raccordé à un circuit sanguin extracorporel, dans lequel le circuit sanguin extracorporel dispose d'un échangeur de chaleur, qui est traversé sur un côté par un flux de sang et sur l'autre côté par un flux de milieu caloporteur et dans lequel la température côté entrée ($T_{di}$) du milieu caloporteur est mesurée à l'entrée de l'échangeur de chaleur et la température côté sortie ($T_{do}$) du milieu caloporteur est mesurée à la sortie de l'échangeur de chaleur et le flux volumique du milieu caloporteur ($Q_d$) est également mesuré,
**caractérisé en ce que**
la température côté entrée ($T_{bi}$) du sang est définie à l'entrée de l'échangeur de chaleur selon la relation

$$T_{bi} = T_{di} + (Q_d/D)\,(T_{do} - T_{di}) \qquad (1)$$

dans lequel la grandeur D est une grandeur caractéristique du transfert de chaleur à travers l'échangeur de chaleur.

2. Procédé selon la revendication 1, **caractérisé en ce que** la grandeur D est déterminée pour une grandeur carac-

téristique $k_0$ A connue de l'échangeur de chaleur à partir de la relation

$$D = Q_b (e^\gamma - 1)/(e^\gamma - (Q_b/Q_d)) \qquad (2)$$

avec $\gamma = k_0$ A $(Q_d - Q_b)/(Q_d Q_b)$
ou

$$D = Q_b (1 - e^{-\gamma})/(1 + (Q_b/Q_d)) \qquad (3)$$

 avec $\gamma = k_0$ A $(Q_d + Q_b)/(Q_d Q_b)$
dans lequel la formule (2) s'applique pour l'écoulement traversant l'échangeur de chaleur à contre-courant et la formule (3) s'applique pour l'écoulement traversant l'échangeur de chaleur dans le sens du courant et dans lequel la grandeur $Q_b$ constitue le flux volumique du sang à travers l'échangeur de chaleur.

3. Procédé selon l'une quelconque des revendications précédentes, **caractérisé en ce que** la grandeur D est déterminée à partir de la relation

$$D = f\, Qb \qquad (5)$$

dans lequel il est prévu que la valeur f est de l'ordre de 1 ou se situe dans la fourchette de $1 \pm 0{,}1$ à $1 \pm 0{,}2$.

4. Procédé selon l'une quelconque des revendications précédentes, **caractérisé en ce que** la température du sang est mesurée dans le circuit extracorporel et une relation entre la valeur mesurée ($T_{art}$) et la valeur définie selon la formule (1) est établie par une régression linéaire, à savoir selon la relation

$$T_{art} = a\, T_{bi} + b \qquad (6)$$

dans lequel il est prévu par ailleurs que $T_{art}$ est calculée à partir de l'équation de régression et que la température corporelle est définie selon la relation

$$T_{Körper} = T_{Umgebung} + (T_{art} - T_{Umgebung})\, \exp(\alpha L/Q_b) \qquad (7)$$

dans lequel $\alpha$ est la conductivité thermique par unité de longueur du tronçon de tuyau flexible du circuit extracorporel entre l'accès vasculaire artériel (1) et l'échangeur de chaleur et L est la longueur dudit tronçon de tuyau flexible.

5. Procédé selon l'une quelconque des revendications précédentes, **caractérisé en ce que** la température corporelle ou une valeur en corrélation avec celle-ci, la différence de celles-ci par rapport à une valeur de référence ou la vitesse de modification de celles-ci est surveillée, dans lequel il est prévu qu'en cas de dépassement ou de non-atteinte d'une valeur limite ou d'une plage de valeurs limites d'une des grandeurs susmentionnées, une alarme est émise.

6. Dispositif servant à définir la température corporelle ou une température en corrélation avec celle-ci d'un patient avec un circuit sanguin extracorporel, dans lequel le circuit sanguin extracorporel dispose d'un échangeur de chaleur, qui est traversé sur un côté par un flux de sang et sur l'autre côté par un flux de milieu caloporteur, et dans lequel le dispositif présente des sondes de mesure, qui sont disposées pour mesurer la température côté entrée ($T_{di}$) du milieu caloporteur à l'entrée de l'échangeur de chaleur ainsi que la température côté sortie ($T_{do}$) du milieu caloporteur à la sortie de l'échangeur de chaleur ainsi que le flux volumique du milieu caloporteur ($Q_d$),
**caractérisé en ce**
**que** le dispositif présente une unité de calcul, qui est réalisée pour définir la température côté entrée ($T_{bi}$) du sang à l'entrée de l'échangeur de chaleur selon la relation

$$T_{bi} = T_{di} + (Q_d/D)\,(T_{do} - T_{di}) \qquad (1)$$

dans lequel la grandeur D est une grandeur caractéristique du transfert de chaleur à travers l'échangeur de chaleur.

**7.** Dispositif selon la revendication 6, **caractérisé en ce que** l'échangeur de chaleur est un dialyseur (5), et que la température en corrélation avec la température corporelle est la température du sang dans le circuit extracorporel.

**8.** Dispositif selon l'une quelconque des revendications 6 ou 7, **caractérisé en ce que** le dispositif présente des moyens de calcul, qui sont réalisés pour déterminer la grandeur D lorsque la grandeur caractéristique $k_0\,A$ de l'échangeur de chaleur est connue à partir de la relation

$$D = Q_b\,(e^{\gamma} - 1)/(e^{\gamma} - (Q_b/Q_d)) \qquad (2)$$

avec $\gamma = k_0\,A\,(Q_d - Q_b)/(Q_d Q_b)$
ou

$$D = Q_b\,(1 - e^{-\gamma})/(1 + (Q_b/Q_d)) \qquad (3)$$

avec $\gamma = k_0\,A\,(Q_d + Q_b)/(Q_d Q_b)$
dans lequel la formule (2) s'applique pour l'écoulement traversant l'échangeur de chaleur à contre-courant et la formule (3) s'applique pour l'écoulement traversant l'échangeur de chaleur dans le sens du courant et dans lequel la grandeur $Q_b$ constitue le flux volumique du sang à travers l'échangeur de chaleur.

**9.** Dispositif selon l'une quelconque des revendications précédentes 6 à 8, **caractérisé en ce que** le dispositif présente des moyens de calcul servant à déterminer la grandeur D, qui sont réalisés pour effectuer sur le côté du milieu caloporteur une variation de température et pour intégrer l'évolution dans le temps de la température du milieu caloporteur à l'entrée et à la sortie sur le temps et pour définir à partir de la relation

$$D = Q_d\,(1 - A_{do}/A_{di}) \quad (4)$$

la grandeur D, dans lequel $A_{do}$ constitue la surface corrigée éventuellement par une ligne de base sous la courbe de température sur le temps au niveau du côté de sortie de l'échangeur de chaleur et $A_{di}$ constitue la surface corrigée par une ligne de base sous la courbe de température sur le temps au niveau du côté d'entrée de l'échangeur de chaleur.

**10.** Dispositif selon l'une quelconque des revendications 6 à 9, **caractérisé en ce que** le dispositif présente des moyens de calcul, qui sont réalisés pour déterminer la grandeur D à partir de la relation

$$D = f\,Q_b \qquad (5),$$

dans lequel il est prévu que la valeur f est de l'ordre de 1 ou se situe dans la fourchette de $1 \pm 0,1$ ou $1 \pm 0,2$.

**11.** Dispositif selon l'une quelconque des revendications 6 à 10, **caractérisé en ce que** le dispositif présente des moyens de surveillance, qui sont réalisés pour surveiller la température corporelle ou une valeur en corrélation avec celle-ci, différence de celles-ci par rapport à une valeur de référence ou la vitesse de modification de celles-ci, dans lequel il est prévu qu'en cas de dépassement ou de non-atteinte d'une valeur limite ou d'une plage de valeurs limites d'une des valeurs susmentionnées, une alarme est émise.

**12.** Dispositif selon l'une quelconque des revendications 6 à 11, **caractérisé en ce que** le dispositif est réalisé pour définir à partir d'une valeur calculée ou connue pour $T_{art}$ la température corporelle selon la relation

$$T_{Körper} = T_{Umgebung} - (T_{art} - T_{Umgebung}) \exp(\alpha L/Qb) \ (7)$$

dans lequel $\alpha$ est la conductivité thermique par unité de longueur du tronçon de tuyau flexible du circuit extracorporel entre l'accès vasculaire artériel (1) et l'échangeur de chaleur et L est la longueur dudit tronçon de tuyau flexible.

**13.** Appareil de traitement du sang comprenant un dispositif selon l'une quelconque des revendications 6 à 12.

**14.** Appareil de traitement du sang selon la revendication 13, **caractérisé en ce que** l'appareil de traitement du sang est un appareil de dialyse et l'échangeur de chaleur du dispositif est un dialyseur (5).

**15.** Appareil de traitement du sang selon la revendication 14, **caractérisé en ce que** le dispositif présente une unité de régulation (9), qui est réalisée pour réguler la température corporelle d'un patient traité avec l'appareil de dialyse ou une température en corrélation avec celle-ci sur une valeur de consigne ou dans une plage de valeurs de consigne, dans lequel l'unité de régulation (9) présente une entrée pour la température calculée en tant que valeur réelle et est réalisée pour comparer celle-ci à une valeur théorique ou une plage de valeurs théoriques, et dans lequel l'unité de régulation (9) est reliée à une unité de thermorégulation (8), qui est réalisée pour modifier la température, faisant office de grandeur de réglage, d'un dialysat traversant le dialyseur (5) dans la mesure où la valeur réelle ne correspond pas à la valeur théorique ou ne se situe pas dans la plage de valeurs théoriques.

Fig. 1

Fig. 2

## Fig.3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2016025268 A2
- EP 0265795 A2
- EP 0773035 A2
- WO 03055544 A1

**In der Beschreibung aufgeführte Nicht-Patentliteratur**

- Replacement of Renal Function by Dialysis
- Principles and Biophysics of Dialysis. Sargent & Gotch. 69-74
- **C. RONCO et al.** Hemodialysis Fluid Composition. 263-266